# EUROPÄISCHE PATENTSCHRIFT

(11) **EP 2 200 603 B1**
(45) Veröffentlichungstag und Bekanntmachung des Hinweises auf die Patenterteilung: **29.06.2011**
(21) Anmeldenummer: 08801690.2
(22) Anmeldetag: 25.08.2008
(51) Int. Cl.: A61K 31/185, A61K 31/205, A61K 31/505, A61K 31/66, A61P 11/00

(54) **OSMOLYTE ZUR BEHANDLUNG VON ALLERGISCH BEDINGTEN ATEMWEGSERKRANKUNGEN**
OSMOLYTES FOR THE TREATMENT OF ALLERGIC RESPIRATORY DISEASES
OSMOLYTES UTILISÉS POUR TRAITER DES AFFECTIONS DES VOIES RESPIRATOIRES D'ORIGINE ALLERGIQUE

(30) Priorität: 27.08.2007 DE 102007040615
(43) Veröffentlichungstag der Anmeldung: 30.06.2010
(73) Patentinhaber: Bitop AG, 58453 Witten (DE)
(72) Erfinder: KRUTMANN, Jean, 41844 Wegberg (DE); LENTZEN, Georg, 58313 Herdecke (DE); SCHWARZ, Thomas, 74072 Heilbronn (DE)
(74) Vertreter: Schöneborn, Holger
(86) Internationale Anmeldenummer: PCT/EP2008/006959
(87) Internationale Veröffentlichungsnummer: WO 2009/027069

(56) Entgegenhaltungen:
- WO-A-00/76528
- WO-A-2005/002556
- WO-A-2005/002581
- WO-A-2007/146869
- WO-A-2007/146870
- WO-A-2008/031028
- WO-A-2008/064916
- CN-A- 1 813 849
- FR-A- 2 674 434
- US-A1- 2001 056 068
- VOLKOV V T: "027-PA11 The estimation of taurine, clycine and B6 influence to bronchial asthma" TUBERCLE AND LUNG DISEASE, CHURCHILL LIVINGSTONE MEDICAL JOURNALS, EDINBURGH, GB, Bd. 76, 1. Oktober 1995 (1995-10-01), Seite 59, XP004539717 ISSN: 0962-8479

## Beschreibung

Osmolyte aus extremophilen Mikroorganismen bilden eine bekannte Gruppe niedermolekularer Schutzstoffe. Extremophile sind sehr außergewöhnliche Mikroorganismen, da sie optimal z.B. bei hohen Salzkonzentrationen (bis 200 g NaCI/I) und hohen Temperaturen (60-110°C) wachsen, die bei mesophilen ("normalen") Organismen zu massiven Schädigungen zellulärer Strukturen führen würden. In den letzten Jahren wurde daher ein großer Forschungsaufwand betrieben, um die biochemischen Komponenten zu identifizieren, die zu der bemerkenswerten thermischen, chemischen und physikalischen Stabilisierung der Zellstrukturen führen. Obwohl viele Enzyme aus hyperthermophilen Mikroorganismen auch unter hohen Temperaturen stabil sind, gilt dies nicht generell für die zellulären Strukturen thermo- und hyperthermophiler Organismen. Zu der hohen Temperaturstabilität von Zellstrukturen tragen in erheblichem Maß niedermolekulare organische Substanzen (Kompatible Solute, Osmolyte) im intrazellulären Milieu bei. Verschiedene neuartige Osmolyte konnten in den letzten Jahren in extremophilen Mikroorganismen erstmals identifiziert werden. In einigen Fällen konnte der Beitrag dieser Verbindungen zum Schutz zellulärer Strukturen gegenüber Hitze und Trockenheit bereits gezeigt werden (Lippert, K., Galinski, E.A. (1994), Appl. Microbiol. Biotech. 37, 61-65; Louis, P., Trüper, H.G., Galinski, E.A. (1994), Appl. Microbiol. Biotech. 41, 684-688; Ramos, Raven, Sharp, Bartolucci, Rossi, Cannio, Lebbink, v. d. Oost, de Vos, Santos (1997), Appl. Environm. Microbiol. 63, 4020-4025; Da Costa, Santos, Galinski (1998), Adv. In Biochemical Engineering Biotechnology, 61, 117-153).

Die in den extremophilen Mikroorganismen gefundenen Osmolyte (kompatible Solute) werden nicht von menschlichen oder tierischen Zellen gebildet.

### Allergische Atemwegserkrankungen

Weltweit nimmt die Anzahl der allergischen Erkrankungen stark zu. Studien haben ergeben, dass weltweit durchschnittlich 7,5% aller Kinder und Jugendlichen an Rhinokonjunktivitis (Heuschnupfen allergischer Natur, kombiniert mit einer Augensymptomatik) leiden (Worldwide variation in prevalence of symptoms of asthma, allergic rhinoconjunctivitis and atopic eczema: ISAAC, Lancet, 351, 1225-1332, 1998). Trotz intensiver Forschungsaktivitäten ist die Pathogenese der Rhinokonjunktivitis immer noch nicht vollständig geklärt. Auch wenn in den vergangenen Jahren deutliche Fortschritte in der Medikamentenbehandlung dieser Erkrankung erzielt wurden, ist die Therapie immer noch nicht zufriedenstellend. Die akuten Symptome (Juckreiz, Reizung, Schwellung, Nasen- bzw. Tränenfluss) der Rhinokonjunktivitis können u. a. mit Hilfe von Antihistaminika gut beherrscht werden. Jedoch haben sie kaum einen therapeutisch relevanten Einfluss auf die der Erkrankung zugrunde liegende und stets fortschreitende Entzündung. Diese Entzündung ist eine Abwehrreaktion des Organismus und seiner Gewebe gegen schädliche Reize mit dem Ziel, den Schaden zu beheben oder zumindest lokal zu begrenzen sowie die Schadensursache (z. B. eingedrungene Bakterien oder Fremdkörper) zu beseitigen. Auslöser einer Entzündung können Mikroorganismen (Bakterien, Viren, Pilze oder Parasiten), Fremdkörper (Pollen, Asbest- oder Silicatkristalle), Gewebezerstörung durch mechanische Schädigung, chemische Noxen und physikalische Einflüsse sowie durch körpereigene Auslöser (zerfallende Tumorzellen, extravasales Blut, Autoimmunreaktionen) oder Kristalle von im Körper ausgefällten Stoffen (Harnsäure, Calciumoxalat und -phosphat, Cholesterin) sein. Durch die Einwirkung der Noxen kommt es im Körper mit Hilfe der T-Helferzellen zu der Freisetzung von Entzündungsmediatoren, insbesondere Histamin nebst Interieukin-8, Leukotrienen und Tumornekrosefaktor-alpha (TNF-alpha) wodurch die nachgeschaltete Kaskade der Entzündungsbekämpfung im Körper aktiviert wird. Im Laufe der Belastung durch die oben genannten Noxen entsteht auch ein Einfluss auf die Adhäsionsmoleküle der durch den äußeren Einfluss betroffenen Epithelien, diese werden, bedingt durch die Noxe direkt oder die körpereigene Reaktion stärker oder weniger stark produziert. Beispielsweise wird durch die Belastung das Molekül ICAM-1 stärker in den betroffenen Zellen exprimiert.

### Allergische Rhinitis und Asthma

Allergene in der Atemluft lösen Reaktionen im Respirationstrakt, typischerweise mit Schleimhautödem und Hypersekretion (allergische Rhinitis, Heuschnupfen) sowie Bronchospasmus (Asthma) aus. Nahrungsallergene dagegen in erster Linie Magen-Darm-Symptome wie Übelkeit, Erbrechen und Durchfall. Die Haut reagiert auf Allergene mit Jucken, Schwellung und Nesselsucht (Urtikaria) sowie atopischer Dermatitis (Neurodermitis). Gelangt das Allergen dagegen direkt in die Blutbahn (z.B. Infusion von Blutprodukten, Medikamente) oder ist die Allergenexposition besonders stark, resultiert eine systemische Sofortreaktion, die unter Umständen einen lebensbedrohlichen Blutdruckabfall nach sich zieht (anaphylaktischer Schock). Die Wirkung der Osmolyte (insbesondere des Ectoins) zur Anwendung bei atopischer Dermatitis und bei Entzündungen des Magen-Darm-Traktes sind in der Deutschen Patentanmeldung DE 103 30 243.3 (Osmolyte zur Behandlung der Neurodermitis) und der Deutschen Patentanmeldung DE 102005011 442.3 (Kompatible Solute enthaltende Mittel zur oralen Verwendung) beschrieben und Stand der Technik.

Die Behandlung von akuten und/oder chronischen Entzündungen der Nasenhaupthöhle und/oder der Nasennebenhöhlen wird vornehmlich mit abschwellenden Nasensprays, kortisonhaltigen Nasensprays, schleimlösenden Substanzen oder Antibiotika durchgeführt. Alle genannten Substanzen sind mit Nebenwirkungen belastet. Kortisonhaltige Nasensprays lösen oft selber allergische Reaktionen aus.

Oft wird die allergische Rhinitis (Rhinokonjunktivitis) sowohl von Patienten als auch vom Arzt als eine Bagatellerkrankung angesehen und dementsprechend nur unzureichend behandelt. In der Folge kann es jedoch zu einem sog. Etagenwechsel kommen, d.h. aus der relativ harmlosen Rhinitis entwickelt sich das sehr ernst zu nehmende Asthma bronchiale. Aus diesem Grunde ist es unerlässlich, bereits die allergische Rhinokonjunktivitis ausreichend und intensiv zu behandeln bzw. ihr vorzubeugen. Nur dann können die Patienten beschwerdefrei leben und nur dann kann ein unter Umständen lebensbedrohlicher Etagenwechsel verhindert werden.

Zum gegenwärtigen Zeitpunkt können die Corticosteroide die der Rhinokonjunktivitis zugrunde liegende Entzündung am wirksamsten bekämpfen. Viele Patienten aber auch Ärzte setzen jedoch diese Medikamente wegen ihrer möglichen systemischen Nebenwirkungen (z.B. Wachstumsverlangsamung, Osteoporose) überhaupt nicht oder nur sehr zögernd, meistens erst in einer späten Phase der Erkrankung ein.

Antihistaminika werden in der akuten Phase der allergischen Rhinokonjunktivitis zur Linderung der oft quälenden Symptome eingesetzt. Jedoch hat die systemische Gabe von Antistaminika trotz der Entwicklung neuer Antihistaminika in Abhängigkeit vom Anwender sedierende Nebenwirkungen (Fahruntüchtigkeit, Müdigkeit), welche den Einsatz deutlich einschränken. Die Präparate der neuen Generation haben zwar deutlich weniger Nebenwirkungen, wirken aber auch in vielen Fällen deutlich schlechter.

Überraschenderweise wurde nun gefunden, dass die Anwendung von Osmolyten, wie z.B. Ectoin, in einer z.B. nasal zu applizierenden Form zur Prophylaxe und Behandlung der "Rhinits allergica (Heuschnupfen)" vorteilhaft ist. Die Rationale basiert auf der Beobachtung, dass es in den Nasenepithelzellen im Rahmen der Entzündungsreaktion, die für die Rhinitis allergica (Heuschnupfen) typisch ist, durch die Interaktion der Epithelzellen mit dem relevanten Allergen (z.B. Pollen) zu einer Aufregulation von Adhäsionsmolekülen, wie z.B. ICAM-1, in diesen Zellen kommt, die die Voraussetzung für die Ausbildung der klinischen Symptome des Schnupfens ist. Von den Erfindern wurde beobachtet, dass sich die Aufregulation des ICAM-1 durch proentzündliche Stimuli durch Ectoin hemmen lässt. Somit kann eine osmolythaltige nasale Applikationsform zur Behandlung und Prophylaxe des Heuschnupfens eingesetzt werden. Aufgrund der ausschließlich wasserstrukturverändernden physikalischen Wirkung der Osmolyte ist praktisch nicht mit den typischen steroidspezifischen Nebenwirkungen zu rechnen.

Überraschenderweise wurde zudem gefunden, dass die topische Verabreichung mit Hilfe eines Nasensprays auf den Nasenepithelien aus verschiedenen Gründen vorteilhaft ist. Die Verabreichung kann dabei simultan, sequentiell oder separat erfolgen. Die topische Verabreichung des osmolythaltigen Nasensprays sorgt für schnelle Beseitigung der akuten Symptome (z.B. Reizung, Juckreiz, Schwellung), ohne das Nebenwirkungen auftreten. Mit dem in der Zubereitung enthaltenem Osmolyt kann die dem Krankheitsbild zugrunde liegende Entzündung erfolgreich bekämpft werden. Häufig ist von dem behandelnden Arzt in Grenzfällen nicht mit letzter Sicherheit festzustellen, ob noch "nur" eine Rhinokonjunktivitis oder bereits eine Atemwegserkrankung, wie Asthma

bronchiale, vorliegt. Vorteilhaft ist, dass die erfindungsgemäße Kombination auch zur Behandlung von Erkrankungen der oberen und unteren Atemwege eingesetzt werden kann.

Überraschenderweise wurde zusätzlich gefunden, dass die Nebenwirkungen wirkstoffhaltiger Nasensprays (z.B., aber nicht ausschließlich, Glucocorticoide, Antihistaminika) durch die Beimischung von Osmolyten deutlich reduziert werden können. Hierbei könnten die Osmolyte mit anderen Wirkprinzipien kombiniert angewendet werden und reduzieren so die notwendige Einsatzkonzentration des anderen Stoffes. Eine andere Möglichkeit wäre die präventive Gabe von Osmolyten, welche direkt das Nebenwirkunsprofil der später zu gebenden Stoffe schwächen. Dies macht die generelle Kombination von Extremolyten mit Steroiden interessant. Somit ist auch eine Kombinationstherapie von Osmolyten (insbesondere Ectoin und Hydroxyectoin) mit anderen intranasal oder intraocular verabreichten Wirkstoffen, die unerwünschte Nebenwirkungen verursachen, sinnvoll. Hierbei ist auch vorstellbar, Osmolyten Wirkstoffformulierungen beizumischen, die nasal oder ocular zur Therapie einer Organkrankheit (z.B. Krebs) verabreicht werden, aber eine entzündliche Nebenwirkung auf das Nasenepithel entfalten. Die gemeinsame Gabe von Osmolyten mit z.B. Glucocorticoiden oder Antihistaminika bzw. die präventive Gabe von Osmolyten vor einer Therapie mit diesen Stoffen könnte als allgemeines Prinzip auch in anderen Applikationsformen (topisch, dermal, intraperitoneal, intravenös, intramuskulär, oral) eine effektive Möglichkeit sein, die Nebenwirkungen der Stoffe zu senken oder durch die Kombination der Wirkprinzipien von Osmolyten mit denen der gleichzeitig oder danach applizierten Wirkstoffe die Einsatzkonzentration der Wirkstoffe zu senken, wodurch eine geringe Belastung des behandelten Patienten entsteht.

Bei den kompatiblen Soluten (Osmolyten) handelt es sich bevorzugt um Ectoin, 4,5,6,7-Tetrahydro-2-methyl-1H-[1,3]-diazepin-4-S-carbonsäure (Homoectoin), Hydroxyectoin, Di-myo-Inositolphosphat (DIP), cyclisches 2,3-Diphosphoglycerat (cDPG), 1,1-Di-Glycerinphosphat (DGP), ß-Mannosylglycerat (Firoin), ß-Mannosylglyceramid (Firoin-A), Di-mannosyl-di-inositolphosphat (DMIP), Glucosylglycerin, Taurin, Betain, Citrullin und/oder ein Derivat dieser Verbindungen wie Salze, Ester oder Säuren. Die Konzentration der kompatiblen Solute liegt typischerweise zwischen 0,01 und 20 Gew.-%, bevorzugt zwischen 0,1 und 10 Gew.-%, besonders bevorzugt zwischen 0,1 und 5 Gew.%, bezogen auf das Gesamtgewicht.

Durch eine auf die jeweilige Behandlung, Applikation und Indikation abgestimmte Verabreichung der Osmolyte können die wirksamen Konzentrationen bereits bei niedrigen Dosierungen erreicht werden. Die Gabe des Osmolyts ermöglicht die Bekämpfung der lästigen Reaktionen wie Juckreiz, Nasenfluss und verhindert das Fortschreiten der Entzündung. Somit ist eine bessere Compliance der Patienten zu erwarten.

Insbesondere bei der intranasalen oder intraocularen Verabreichung kommt es nicht nur zu einer schnellen Wirkung, sondern auch zu einer hohen therapeutischen Wirksamkeit, die mit einer starken antientzündlichen Wirkung einhergeht. Der vorliegenden Erfindung liegt daher die Aufgabe zugrunde, Mittel zur Prävention oder Therapie von immunologischen Überempfindlichkeitsreaktionen, insbesondere der allergischen Rhinitis zur Verfügung zu stellen, die einen breiteren (bewusst nicht monospezifischen), nebenwirkungsarmen und damit einen effektiveren Therapieansatz darstellen. Ein Aspekt der vorliegenden Erfindung besteht in der Verwendung von Osmolyten zur Herstellung eines Arzneimittels oder Medizinproduktes z.B. in Form eines Nasensprays oder in Form von Augentropfen zur Prävention oder Therapie der allergischen Rhinitis, indem es durch den osmolytvermittelten Schutz der Nasenepithelzellen zu einer Abschwächung der Expression pro-inflammatorischer Genprodukte (z.B. ICAM1) im Rahmen von Entzündungsreaktionen kommt.

Die Dosierung kann mehrmals täglich erfolgen, wobei die Einzeldosis von dem verwendeten Osmolyten und insbesondere vom allgemeinen Zustand des Patienten (Alter, Gewicht, etc.) und dem Schweregrad der Erkrankung abhängt.

Für die topische Anwendung können verschiedene pharmazeutische Formulierungen, z.B. Nasensprays, Nasentropfen und Augentropfen, in Frage kommen. Aufgrund der Wasserlöslichkeit des Wirkstoffes Osmolyt (bevorzugt Ectoin) können Formulierungen mit diesen Wirkstoffen vorzugsweise als wäßrige Lösungen formuliert werden.

Neben den wirksamen Osmolyten können die erfindungsgemäßen pharmazeutischen Zubereitungen weitere Bestandteile wie Konservierungsstoffe, Stabilisatoren, Isotonisierungsmittel, Verdickungsmittel, Suspensionsstabilisatoren, Hilfstoffe zur pH-Einstellung, Puffersysteme und Netzmittel enthalten. Die erfindungsgemäßen pharmazeutischen Zubereitungen können zudem weitere wirksame Bestandteile wie Antihistaminika oder Steroidwirkstoffe (z. B. Loteprednoletabonat) enthalten.

Als Konservierungsmittel kommen in Frage: Benzalkoniumchlorid, Chlorbutanol, Thiomersal, Methylparaben, Propylparaben, Sorbinsäure und deren Salze, Natriumedetat, Phenylethyl-alkohol, Chlohexidinhydrochloridacetat, -digluconat, Cetylpyridiniumchlorid, -bromid, Chlorkresol, Phenylquecksilberacetat, Phenylquecksilbernitrat, Phenyquecksilberborat, Phenoxyethanol.

Geeignete Hilfsstoffe zur Einstellung der Isotonie der Formulierungen sind beispielsweise: Natriumchlorid, Kaliumchlorid, Mannitol, Glucose, Sorbitol, Glycerol, Propylenglycol. Im Allgemeinen werden diese Hilfsstoffe in Konzentrationen von 0,1 bis 10% eingesetzt.

Die Formulierungen der Erfindung können ebenfalls geeignete Puffersysteme oder andere Hilfsstoffe zur pH-Einstellung beeinhalten, um einen pH-Wert einzustellen und aufrechtzuerhalten in der Größenordnung von 4-8, vorzugsweise von 5 bis 7,5. Geeignete Puffersysteme sind Citrat, Phosphat, Tromethamol, Glycin, Borat, Acetat. Diese Puffersysteme können hergestellt werden aus Substanzen wie, Citronensäure, Mononatriumphosphat, Dinatriumphosphat, Glycin, Borsäure, Natriumtetraborat, Essigsäure oder Natriumactat.

Es können ebenfalls weitere Hilfsstoffe zur pH-Einstellung verwendet werden wie Salzsäure oder Natriumhydroxid. Als Netzmittel für die Formulierungen kommen in Frage: Benzalkoniumchlorid, Cetylpyridiniumchlorid, Tyloxapol, verschiedene Polysorbate [Tween^{(™)}] sowie weitere polyoxyethylierte Substanzen und Poloxamere.

Das nachfolgende Beispiel eines Nasensprays illustriert die Erfindung

### Nasenspray mit Ectoin (0,1 %)

In einem geeigneten Rührwerksbehälter ca. 45 kg gereinigtes Wasser vorlegen. Darin den Wirkstoff Ectoin, Hydroxypropylmethylcellulose, Natriumacetat, Benzalkoniumchlorid und Sorbitollösung nacheinander zugeben und unter Rühren auflösen. Die entstandene Lösung mit gereinigtem Wasser auf ein Volumen von 49,5 Liter auffüllen. Den pH-Wert der Lösung mit 1 N Natronlauge auf pH 6,0 einstellen. Mit gereinigtem Wasser auf das Endvolumen von 50,0 Liter auffüllen und rühren. Die Lösung durch einen geeigneten Filter filtrieren und in Flaschen abfüllen, welche anschließend mit einer geeigneten Nasenspraypumpe versehen werden.

### Wirksamkeitsstudien

### Beispiel 1

### Behandlung der Sensibilisierung und Induktion von allergischen Reaktionen bei Mäusen mit Ovalbumin (OVA)

Die Sensibilisierung von sieben Wochen alten Mäusen des Inzuchtstammes Balb/c erfolgte durch intraperitoneale Injektion eines Gemisches von Ovalbumin und Aluminiumhydroxid in einem Gesamtvolumen von 200 µl Puffer (PBS). Insgesamt erfolgt die Injektion zweimal, am Tag 0 und am Tag 14. Die Gruppengröße pro untersuchter Gruppe betrug n=8.

Um anschließend eine allergische Reaktion in der Lunge der Mäuse hervorzurufen, also das akute allergische Asthma auszulösen, wurden die Mäuse am Tag 28 und Tag 38 jeweils 30 Minuten lang mit Ovalbumin-Aerosol inhalativ behandelt. Hierzu wurden die Mäuse in eine Plexiglas-Kammer gesetzt. In diesen wurde über einen Inhalator eine 1% OVA-Lösung vernebelt eingegeben.

### Behandlung der Tiere mit Ectoin-Lösung oder 0,9% Salz-Lösung

Zur Behandlung der Tiere mit Ectoin werden die Tiere mittels einer Mischung aus Ketamin und Rompun narkotisiert. 50 µl einer sterilen Ectoin Lösung oder 0,9% Salzlösung wurde dann mittels Glaskapillare vor die Nasenöffnung appliziert, bis diese vollständig inhaliert wurde. Die insgesamt 14 Behandlungen begannen am Tag 0. Die letzte Behandlung erfolgte an Tag 32.

### Ergebnis

Als Endpunkte der Studie wurden die bronchiale Hyperreaktivität mittels Plethysmographen, die zelluläre Zusammensetzung der Broncho-Alveolären-Lavage (BAL) und die Freisetzung von OVA-spezifischen IgE und IgG1 Antikörpern bestimmt.

Ein positiver Einfluß der Behandlung mit Ectoin-Lösung im Vergleich zur Behandlung mit 0,9% Salzlösung auf diese Endpunkte konnte eindeutig festgestellt werden.

### Beispiel 2

### Sensibilisierung und Induktion von allergischem Asthma bei Ratten mit Ovalbumin (OVA)

In der Prüfung wurden 4 Behandlungsgruppen, eine Referenzgruppe, eine scheinsensibilisierte und Vehikel-behandelte Negativkontrollgruppe und eine Vehikelbehandelte Positivkontrollgruppe eingesetzt. Die Gruppengröße betrug n=16.

Im ersten Schritt der Prüfung wurden alle Tiere der Gruppen bis auf die scheinsensibilisierte Gruppe gegenüber Ovalbumin (Ova) plus Adjuvantien systemisch sensibilisiert, die Negativkontrolle erhielt NaCl. An Tag 7 und 14 wurden die Tiere intratracheal mit Ova geboostert (Verstärkung der Sensibilisierung). 48 h, 24 h und 2 h vor der finalen inhalativen Allergenexposition erfolgte eine intratracheale Vorbehandlung mit der Prüfsubstanz Ectoin in drei Dosierungsstufen in drei Gruppen sowie mit Positivkontrolle. Die beiden Kontrollgruppen erhielten dabei nur NaCl. Nach Ablauf der Wartephase wurden die Tiere dann narkotisiert, schonend orotracheal intubiert und nach Legen eines Oesophaguskatheters und Erreichen eines Steady state zunächst die Lungenfunktion vor Provokation bodyplethysmographisch gemessen (inkl. Parametern wie Atemzugvolumen, Atemfrequenz, dynamischer Compliance und Lungen-resistance). Die Datenspeicherung und -auswertung erfolgten mit einem speziell für diese Lungenfunktionstests entwickelten Rechnerprogramm (HEM, Notocord/Frankreich). Dann erfolgt eine definierte inhalative Exposition der Versuchstiere gegenüber dem Allergen Ovalbumin (Ovalbumin-Challenge, Modell einer allergisch induzierten asthmatischen Reaktion).

### Einfluss der Ectoinlösung auf die allergische Frühreaktion

Während und nach der Exposition wurde die Aufzeichnung der Lungenfunktionsparameter fortgesetzt zur Erfassung des Bronchospasmus.

Es konnte ein eindeutiger positiver Einfluss der Ectoin-Lösung auf die Lungenfunktionsparameter im Vergleich zur Negativkontrolle festgestellt werden.

### Einfluss der Ectoin-lösung auf die allergische Spätreaktion

Die späte allergische Phase wurde 24 h nach Ovalbumin-Challenge untersucht. Zunächst wurden die Tiere auf Atemwegshyperreaktivität gegenüber unspezifischen Stimuli untersucht. Dazu wurde ein Hyperreaktivitätstest in Form einer stufenweise ansteigenden inhalativen Acetylcholinprovokation durchgeführt. Danach wurden die Tiere schmerzlos getötet und die Lungen lavagiert (BAL). Aus der Lavageflüssigkeit wurde die Gesamtzellzahl und das Differentialzellbild inkl. Anzahl der Eosinophilen in der BAL bestimmt.

Ein positiver Einfluß der Behandlung mit Ectoin-Lösung im Vergleich zur Behandlung mit der Negativ-Kontrolle auf die allergische Spätreaktion konnte eindeutig festgestellt werden.

### Beispiel 3

### Einfluss von Ectoin-Nasen-Spray auf die allergische Reaktion im Menschen

In einer randomisierten, doppelblinden Crossover Studie mit 20 Patienten, welche an einer allergischen Rhinitis leiden, wurde der Effekt eines Ectoin-Nasensprays auf die Reaktion nach einem "Allergen-Challenge" untersucht.

Die Patienten wurden zu Beginn der Studie intranasal mit einem definierten Allergen angeregt und die allergische Reaktion wurde anhand verschiedener Parameter vermessen. Daraufhin wurde eine 2-wöchige Behandlung mit Ectoin-Nasenspray oder Placebo-Nasenspray (0,9 % NaCl) durchgeführt und eine erneute Anregung mit dem definierten Allergen nebst Vermessung der allergischen Reaktion durchgeführt.

Nach einer ausreichenden Auswaschphase wurden die Patienten im Crossover analog mit dem Ectoin-Nasenspray oder Placebo-Nasenspray behandelt.

Somit konnte die Wirkung von Ectoin-Nasenspray intra-individuell und interindividuell mit der Placebo-Lösung verglichen werden. Es konnte gezeigt werden, dass das Ectoin-Nasenspray die Symptome der allergischen Rhinitis in dieser Studie im Vergleich zu dem Placebo-Nasenspray deutlich verbesserte.

### Beispiel 4

### Reduktion der ICAM-1 Expression durch DGP

Humane Keratinozyten wurden für 24 h mit DGP vorbehandelt. Nach Bestrahlung mit UVA-Strahlung (Umwelt-Noxe) wurde die Menge an ICAM-1 gemessen. DGP inhibierte die Expression um 49 %.

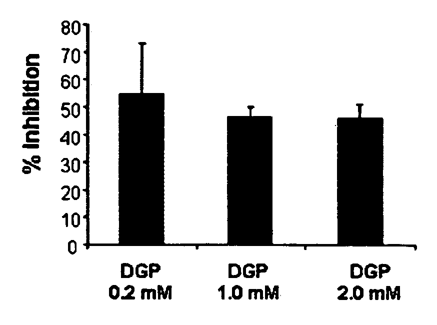

Figur: Inhibition der ICAM-1 Expression durch DGP in verschiedenen Einsatzkonzentrationen

### Beispiel 5

### Hemmung der ICAM-1 Expression durch Ectoin

Die Expression von ICAM-1 wurde mit der differential reverse transcriptase-PCR (RT-PCR) und dem Kit von Applied Biosystem gemessen. Um die normalen Schwankungen in der Genexpression der Hautzellen zu berücksichtigen, wird die ICAM-1 Expression im Verhältnis zu dem konstitutiv gebildeten house-keeping Gen ß-Aktin ins Verhältnis gesetzt. Die semiquantitative Analyse der RT-PCR wurde mit einer Ionenaustauscherchromatographie mit einem UV-Spektrophotometer (A260) durchgeführt. (A) Nicht vorbehandelte, bestrahlte Kontrolle, (B) mit 1 mM RonaCareTMEctoin 24 h vorinkubiert und mit einer Einzeldosis von 30 J/cm² bestrahlt oder (C) mit 1 mM Ectoin 24 h vorinkubiert, unbestrahlt dem Zellmedium 24 h vorinkubiert.. Die UVA-Strahlung induziert eine Aufregulation der ICAM-1-Expression. Die Vorbehandlung von Keratinozyten mit 1 mM Ectoin kann die durch UVA-Strahlung induzierte ICAM-1-Induktion zu allen Zeitpunkten fast vollkommen aufheben.

## Patentansprüche

1. Medikament enthaltend als Wirkstoff mindestens ein kompatibles Solut, ausgewählt aus der Gruppe bestehend aus Ectoin, 4,5,6,7-Tetrahydro-2-methyl-1H-[1,3]-diazepin-4-S-carbonsäure (Homoectoin), Hydroxyectoin, Di-myo-Inositolphosphat (DIP), cyclisches 2,3-Diphosphoglycerat (cDPG), 1,1-Di-Glycerinphosphat (DGP), ß-Mannosylglycerat (Firoin), ß-Mannosylglyceramid (Firoin-A), Di-mannosyl-di-inositolphosphat (DMIP), Glucosylglycerin, Betain und/oder Derivaten dieser Verbindung aus gewicht aus Salzen, Estern oder Säuren, zur Anwendung in der Prävention und/oder Behandlung von allergisch bedingten Atemwegserkrankungen.

2. Medikament zur Anwendung nach Anspruch 1, **dadurch gekennzeichnet, dass** die Atemwegserkrankung Rhinitis allergica oder Asthma ist.

3. Medikament zur Anwendung nach Anspruch 1 oder 2, **dadurch gekennzeichnet, dass** das kompatible Solut in einer Konzentration von 0,01 bis 20 Gew.-%, bevorzugt 0,1 bis 10 Gew.-%, besonders bevorzugt 0,1 bis 5 Gew.-% bezogen auf das Gesamtgewicht vorliegt.

4. Medikament zur Anwendung nach einem der Ansprüche 1 bis 3, **dadurch gekennzeichnet, dass** das Medikament in flüssiger oder halbflüssiger Applikationsform vorliegt.

5. Medikament zur Anwendung nach einem der Ansprüche 1 bis 4, **dadurch gekennzeichnet, dass** das Medikament in Form eines Sprays, in Form von Augentropfen oder Nasentropfen oder als inhalierbare flüssige oder feste Zubereitung vorliegt.

6. Medikament zur Anwendung nach einem der Ansprüche 1 bis 5, **dadurch gekennzeichnet, dass** das Medikament mindestens ein Antihistaminikum oder ein Steroid, insbesondere ein Glucocorticoid, enthält.

7. Medikament zur Anwendung nach einem der Ansprüche 1 bis 6, **dadurch gekennzeichnet, dass** das Medikament mindestens einen Mastzellstabilisator, einen Leukotrienrezeptormodifikator und/oder ein β2-Sympathomimetikum enthält.

## Claims

1. Medicament containing, as the active substance, at least one compatible solute that is chosen from the group of ectoine, 4,5,6,7-tetrahydro-2-methyl-1H-[1,3]-diazepine-4-S-carboxylic acid (homoectoine), hydroxyectoine, di-myo-inositol-phosphate (DIP), cyclic 2,3-diphosphoglycerate (cDPG), 1,1-diglycerol phosphate (DGP), β-mannosylglycerate (firoin), β-mannosylglyceramide (firoin-A), dimannosyl-diinositol phosphate (DMIP), glucosylglycerol, betaine and/or derivatives of these compounds, selected from salts, esters or acids, for use in the prevention and/or treatment of allergic respiratory diseases.

2. Medicament for a use according to claim 1, **characterized in that** the respiratory disease is rhinitis allergica or asthma.

3. Medicament for a use according to claim 1 or 2, **characterized in that** the compatible solute has a concentration of between 0.01 and 20% w/w, preferably between 0.1 and 10% w/w, especially preferably between 0.1 and 5% w/w, related to the total weight.

4. Medicament for a use according to any of claims 1 to 3, **characterized in that** the medicament is available for application in liquid or semi-liquid form.

5. Medicament for a use according to any of claims 1 to 4, **characterized in that** the medicament is available in the form of a spray, as eye drops or nose drops or as an inhalable liquid or solid preparation.

6. Medicament for a use according to any of claims 1 to 5, **characterized in that** the medicament contains at least one antihistamine or steroid, especially a glucocorticoid.

7. Medicament for a use according to any of claims 1 to 6, **characterized in that** the medicament contains at least one mast cell stabilizer, one leukotriene receptor modifier, and/or one β2-sympathomimetic substance.

## Revendications

1. Médicament comprenant comme matière active au moins un soluté compatible, sélectionné dans le groupe consistant en ectoïne, acide 4,5,6,7-tétrahydro-2-méthyl-1H-[1,3]-diazépin-4-S-carbonique (homoectoïne), hydroxy-ectoïne, di-myo-inositolphosphate (DIP), 2,3-diphosphoglycérate cyclique (cDPG), 1,1-diglycérinphosphate (DGP), β-mannosylglycérat (firoïne), β-mannosylglycéramide (firoïne-A), di-mannosyl-di-inositolphosphate (DMIP), glucosylglycérine, bétaïne et/ou des dérivés de ces composés sélectionnés parmi des sels, esters ou acides, pour l'utilisation dans la prévention et/ou le traitement de maladies allergiques des voies respiratoires.

2. Médicament pour utilisation selon la revendication 1, **caractérisé en ce que** la maladie des voies respiratoires est la rhinite allergique ou l'asthme.

3. Médicament pour utilisation selon la revendication 1 ou 2, **caractérisé en ce que** le soluté compatible est présent en une concentration de 0,01 à 20% en poids, de préférence de 0,1 à 10% en poids, selon une plus grande préférence de 0,1 à 5% en poids par rapport au poids total.

4. Médicament pour utilisation selon l'une des revendications 1 à 3, **caractérisé en ce que** le médicament est présent dans une forme d'application liquide ou semi-liquide.

5. Médicament pour utilisation selon l'une des revendications 1 à 4, **caractérisé en ce que** le médicament est présent sous forme d'un spray, sous forme de gouttes pour les yeux ou de gouttes pour le nez ou comme préparation liquide ou solide pouvant être inhalée.

6. Médicament pour utilisation selon l'une des revendications 1 à 5, **caractérisé en ce que** le médicament contient au moins un anti-histaminique ou un stéroïde, en particulier un glucocorticoïde.

7. Médicament pour utilisation selon l'une des revendications 1 à 6, **caractérisé en ce que** le médicament contient un stabilisateur de mastocytes, un modificateur du récepteur des leucotriènes et/ou un β2-sympathomimétique.
